# FASCICULE DE BREVET EUROPEEN

(11) **EP 0 591 060 B1**
(45) Date de publication et mention de la délivrance du brevet: **18.12.1996**
(21) Numéro de dépôt: 93402397.9
(22) Date de dépôt: 01.10.1993
(51) Int. Cl.: C07C 323/36, A61K 7/13, C07D 263/58, C07C 323/20

(54) **Métaaminophénols 4-substitués, leur procédé de préparation, leur utilisation pour la teinture des fibres kératiniques et les composés intermédiaires utilisés dans le procédé de préparation**
4-Substituierte Para-aminophenole, Verfahren zur deren Herstellung und deren Verwendung für die Färbung von Keratinfasern und Zwischenprodukte verwendet während der Herstellung
4-Substituted meta amino phenols, process for their preparation their use in dyeing keratinic fibres and intermediates used in their preparation

(30) Priorité: 02.10.1992 FR 9211712
(43) Date de publication de la demande: 06.04.1994
(73) Titulaire: L'OREAL, 75008 Paris (FR)
(72) Inventeur: Junino, Alex, F-93190 Livry Gargan (FR); LaGrange, Alain, F-77700 Coupvray (FR); Genet, Alain, F-93600 Aulnay-sous-Bois (FR)
(74) Mandataire: Casalonga, Axel

(56) Documents cités:
- EP-A- 0 331 144
- DE-A- 4 017 516
- FR-A- 1 061 331
- FR-A- 2 547 300
- US-A- 3 811 831

## Description

La présente invention a pour objet de nouveaux métaaminophénols soufrés, les produits intermédiaires nécessaires à leur préparation et leur utilisation en teinture des fibres kératiniques, notamment à titre de coupleurs dans des compositions tinctoriales, ainsi que des procédés de teinture mettant en oeuvre ces compositions.

Il est connu de teindre les fibres kératiniques et notamment les cheveux humains, avec des compositions tinctoriales contenant des précurseurs de colorants d'oxydation et des coupleurs.

Les coupleurs encore appelés modificateurs de coloration, permettent de faire varier les nuances obtenues avec les précurseurs de colorants d'oxydation.

Dans le domaine de la teinture des fibres kératiniques et en particulier des cheveux humains, on est à la recherche de coupleurs qui, associés à des précurseurs de colorants d'oxydation, permettent d'obtenir un large éventail de nuances aux reflets variés, tout en conférant aux cheveux une coloration ayant une résistance satisfaisante à la lumière, au lavage, aux intempéries, à la transpiration et aux différents traitements que peuvent subir les cheveux.

La demanderesse a découvert, ce qui fait l'objet de l'invention, que les nouveaux métaaminophénols soufrés définis ci-après, utilisés en tant que coupleurs dans des compositions tinctoriales d'oxydation, permettent d'obtenir un large éventail de nuances aux reflets variés et confèrent aux cheveux une coloration stable ayant une résistance à la lumière, au lavage, aux intempéries, à la transpiration et aux différents traitements que peuvent subir les cheveux, particulièrement remarquable.

La présente invention a donc pour objet de nouveaux métaaminophénols soufrés.

Un autre objet de l'invention est constitué par les produits intermédiaires utilisés pour la préparation de ces métaaminophénols soufrés.

La présente invention a également pour objet l'utilisation pour la teinture des fibres kératiniques, et plus particulièrement des cheveux humains, de ces métaaminophénols.

Un autre objet de l'invention porte sur des compositions tinctoriales d'oxydation, destinées à être utilisées pour la teinture des fibres kératiniques et en particulier des cheveux humains, contenant au moins un précurseur de colorant d'oxydation de type ortho et/ou para et au moins, à titre de coupleur, ces nouveaux métaaminophénols soufrés.

Un autre objet de l'invention est constitué par le procédé de coloration des fibres kératiniques et en particulier des cheveux humains, mettant en oeuvre une telle composition mélangée à un agent oxydant.

D'autres objets de l'invention apparaîtront à la lecture de la description et des exemples qui suivent.

La présente invention a donc pour objet de nouveaux métaaminophénols soufrés de formule générale : 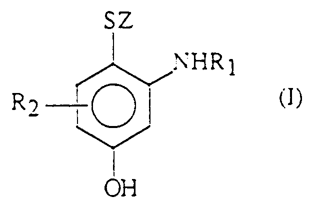
dans laquelle Z représente un radical alkyle en C₁-C₁₈, un radical aralkyle dans lequel le radical alkyle est en C₁-C₆, un radical monohydroxyalkyle en C₁-C₆ ou polyhydroxyalkyle en C₂-C₆, un radical aryle, un radical aminoalkyle de formule : 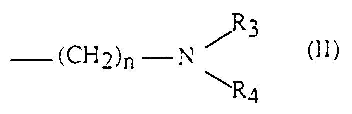
dans laquelle :
n est un nombre entier compris entre 1 et 6 inclus;
R₃ et R₄, identiques ou différents, représentent un atome d'hydrogène ou un radical alkyle en C₁-C₄, hydroxyalkyle en C₁-C₄, acyle en C₁-C₆;
R₁ représente un atome d'hydrogène, un radical alkyle en C₁-C₆, un radical monohydroxyalkyle en C₁-C₆, polyhydroxyalkyle en C₂-C₆, monocarbamylalkyle en C₁-C₆, dicarbamylalkyle en C₁-C₆, aminoalkyle en C₁-C₆, acyl(C₁-C₆)aminoalkyle(C₁-C₄), carbalcoxy (C₂-C₆)alkyle(C₁-C₄), carbamyle ou monoalkyl en C₁-C₆ carbamyle;
R₂ représente un atome d'hydrogène, un radical alkyle en C₁-C₄, un radical monohydroxyalkyle en C₁-C₄, un radical alcoxy en C₁-C₄,
ainsi que leurs sels d'acides.

Parmi les significations préférées du radical Z dans les métaaminophénols soufrés de formule générale (I) selon l'invention, le radical alkyle en C₁-C₁₈ désigne un radical méthyle, éthyle, propyle, butyle, dodécyle, hexadécyle, le radical aralkyle désigne le radical benzyle, le radical aryle désigne le radical phényle, le radical mono-ou
polyhydroxyalkyle est choisi parmi :
-CH₂-CH₂-OH, -CH₂-CHOH-CH₂-OH, -CH₂-CHOH-CH₃ ,
le radical aminoalkyle est choisi parmi :
-CH₂-CH₂NH₂ , -CH₂-CH₂-NHCH₃ ,
le radical acylaminoalkyle est choisi parmi :
-CH₂-CH₂-NH-COCH₃ ou

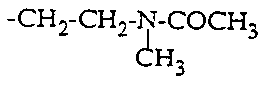

Lorsque les groupements R₃ et/ou R₄ désignent le radical acyle, celui-ci désigne de préférence les radicaux formyle, acétyle et propionyle;

R₂ désigne de préférence un atome d'hydrogène ou un radical alcoxy en C₁-C₄.

Les sels d'acides correspondants sont choisis de préférence parmi les chlorhydrates, les sulfates ou les bromhydrates.

Parmi les métaaminophénols soufrés de formule (I), on peut citer :
le 2-méthoxy 4-méthylthio 5-aminophénol
le 4-méthylthio 3-aminophénol
ou, selon la nomenclature IUPAC :
le 5-amino 2-méthoxy 4-méthylsulfanylphénol
le 3-amino 4-méthylsulfanylphénol.

Les métaaminophénols soufrés de formule (I) et leurs sels cosmétiquement acceptables peuvent être préparés selon un procédé en plusieurs étapes.

Selon un premier procédé et dans une première étape, on fait réagir en présence d'une base telle que la potasse ou le carbonate de potassium, l'halogénure de 2-nitro 4-OR₆-benzène, substitué ou non, sur un thiol de formule (III) :

Z' - SH (III)

dans laquelle Z' représente un radical alkyle en C₁-C₁₈, aralkyle dans lequel le radical alkyle est en C₁-C₆, monohydroxyalkyle en C₁-C₆, polyhydroxyalkyle en C₂-C₆, un radical aryle ou un groupement de formule (IV) : 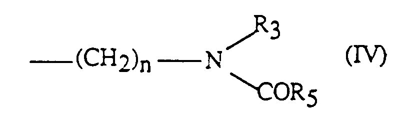
dans laquelle :
R₃ et n ont les significations indiquées précédemment dans la formule (I);
R₅ représente un atome d'hydrogène ou un radical alkyle en C₁-C₃;
R₆ représente un radical alkyle en C₁-C₁₈, aralkyle, silyle, pyranyle, un atome d'hydrogène;
dans une deuxième étape, on réduit le substituant nitro du composé de formule (V) : 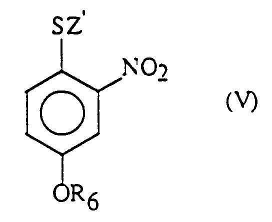
obtenu précédemment pour préparer un composé répondant à la formule (VI) : 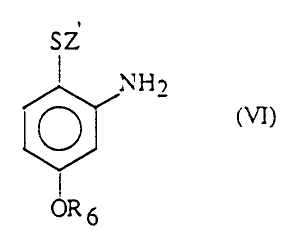
dans laquelle Z' et R₆ ont les significations indiquées ci-dessus; éventuellement, dans une troisième étape, et suivant le composé métaaminophénol soufré de formule (I) que l'on souhaite obtenir, on effectue :
a) soit une déprotection du phénoléther
b) soit une mono-substitution de l'amine aromatique pour obtenir un composé de formule (I) dans laquelle R₁ est différent de H; le groupement-OR₆ peut ensuite être déprotégé,
c) soit une hydrolyse acide du composé (VI) dans lequel Z représente 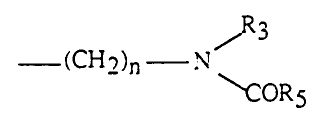 pour obtenir le composé de formule (VII) : 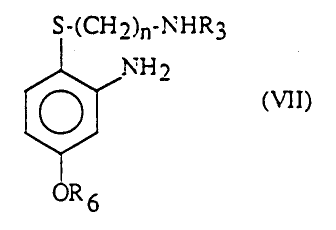 dans laquelle R₃, R₆ et n ont les significations indiquées ci-dessus, R₃ ne désignant toutefois pas de radical acyle en C₁-C₆.
   l'amine nucléaire peut être ensuite monosubstituée, et le groupement -OR₆ déprotégé,
d) soit on effectue au préalable une substitution de l'amine extra-nucléaire sur le composé de formule (VI) dans laquelle Z' représente le groupement (IV), pour obtenir le composé de formule (VIII) : 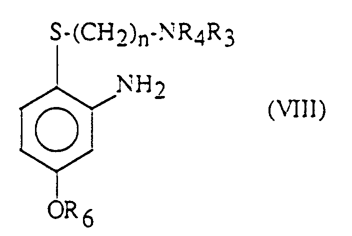 dans laquelle R₃, R₄, R₆ et n ont les significations indiquées ci-dessus, l'amine nucléaire peut être ensuite monosubstituée, et le groupement -OR₆ déprotégé.

Ces étapes peuvent être suivies par une substitution du noyau benzénique par un groupement R₂ ayant la signification indiquée ci-dessus, mais ne désignant toutefois pas un atome d'hydrogène.

Selon un deuxième procédé et dans une première étape, on fait réagir un composé substitué de formule (IX) : 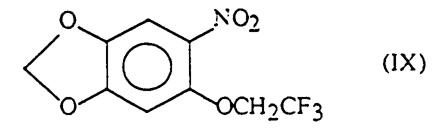
sur un thiol de formule :

Z' - SM (X)

dans laquelle M est un radical alcalin et Z' a les significations indiquées ci-dessus, pour obtenir le composé de formule (XI) :

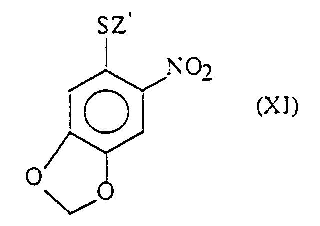

Dans une deuxième étape, on ouvre le cycle dioxolane du composé de formule (XI) par un alcoolate, pour préparer un composé répondant à la formule (XII) :

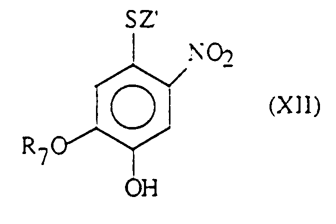

R₇ représente un groupe alkyle en C₁-C₄, linéaire ou ramifié.

La réduction des groupes nitro des composés de formules (V) ou (XII) s'effectue de préférence en utilisant du fer en milieu acétique ou par le cyclohexène en présence d'un catalyseur palladium-charbon, ou encore par la poudre de zinc en présence de chlorure d'ammonium et d'éthanol, ou par tout autre procédé de réduction classique.

La substitution de l'amine aromatique ou de l'amine extra-nucléaire peut être effectuée en faisant réagir, par exemple, le bromure d'éthyle, la bromhydrine du glycol, l'éthyl-chloroformiate, la β-chloracétamide, ou l'anhydride acétique.

L'invention a également pour objet les composés intermédiaires nouveaux de formules (V), (XI) et (XII) définies ci-dessus, utilisables dans la synthèse des métaaminophénols de formule (I).

Un autre objet de l'invention est constitué par l'utilisation pour la teinture des fibres kératiniques et en particulier des cheveux humains, d'au moins un métaaminophénol soufré de formule (I) tel que défini précédemment.

Les composés de formule (I) sont appliqués sur les fibres kératiniques et en particulier les cheveux humains, au moyen de compositions tinctoriales qui constituent un autre objet de l'invention.

Les compositions conformes à l'invention contiennent dans un milieu approprié pour la teinture, au moins un métaaminophénol soufré défini ci-dessus. Les compositions préférées contiennent au moins un métaaminophénol soufré défini ci-dessus à titre de coupleur, en association avec au moins un précurseur de colorant d'oxydation de type ortho et/ou para.

Les précurseurs de colorants d'oxydation de type ortho et/ou para utilisés dans les compositions conformes à l'invention, sont des composés qui ne sont pas des colorants en eux-mêmes, mais qui forment un colorant par un processus de condensation oxydative, soit sur eux-mêmes, soit en présence d'un coupleur ou modificateur.

Ces précurseurs de colorants d'oxydation de type ortho ou para sont des composés benzéniques ou hétérocycliques qui comportent deux groupements fonctionnels amino ou amino et hydroxy, en position ortho ou para l'un par rapport à l'autre.

Les précurseurs de colorants d'oxydation de type ortho ou para peuvent être choisis parmi les paraphénylènediamines, les paraaminophénols, les précurseurs hétérocycliques para dérivés de la pyridine, de la pyrimidine ou du pyrazole, tels que la 2,5-diaminopyridine, la 2-hydroxy 5-aminopyridine, la 2,4,5,6-tétraaminopyrimidine, le 4,5-diamino 1-méthylpyrazole, la 2-diméthylamino 4,5,6-triaminopyrimidine, les orthoaminophénols et les bases dites "doubles".

A titre de paraphénylènediamines, on peut plus particulièrement citer les composés répondant à la formule (XIII) : 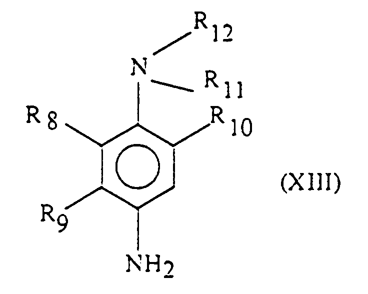
dans laquelle :
R₈, R₉, R₁₀, identiques ou différents, représentent un atome d'hydrogène ou d'halogène, un radical alkyle, un radical alcoxy, un radical carboxy, sulfo ou hydroxyalkyle en C₁-C₄ ;
R₁₁ et R₁₂, identiques ou différents, représentent un atome d'hydrogène, un radical alkyle, hydroxyalkyle, alcoxyalkyle, carbamylalkyle, mésylaminoalkyle, acétylaminoalkyle, uréidoalkyle, carbalcoxyaminoalkyle, sulfoalkyle, pipéridinoalkyle, morpholinoalkyle, ou phényle éventuellement substitué en para par un groupement amino.; ou bien R₁₁ et R₁₂ forment conjointement avec l'atome d'azote auquel ils sont liés, un hétérocycle pipéridino ou morpholino, sous réserve que R₈ ou R₁₀ représente un atome d'hydrogène lorsque R₁₁ et R₁₂ ne représentent pas un atome d'hydrogène, ainsi que les sels de ces composés. Ces radicaux alkyle ou alcoxy ont de préférence 1 à 4 atomes de carbone et désignent notamment les radicaux méthyle, éthyle, propyle, méthoxy et éthoxy.

Parmi les composés de formule (XIII), on peut citer la paraphénylènediamine, la p-toluylènediamine, la méthoxyparaphénylènediamine, la chloroparaphénylènediamine, la 2,3-diméthylparaphénylènediamine, la 2,6-diméthylparaphénylènediamine, la 2,6-diéthylparaphénylènediamine, la 2,5-diméthylparaphénylènediamine, la 2-méthyl 5-méthoxyparaphénylènediamine, la 2,6-diméthyl 5-méthoxyparaphénylènediamine, la N,N-diméthylparaphénylènediamine, la N,N-diéthylparaphénylènediamine, la N,N-dipropylparaphénylènediamine, la 3-méthyl 4-amino N,N-diéthylaniline, la N,N-di-(β-hydroxyéthyl)paraphénylènediamine, la 3-méthyl 4-amino N,N-di-(β-hydroxyéthyl)aniline, la 3-chloro 4-amino N,N-di-(β-hydroxy-éthyl)aniline, la 4-amino N,N-(éthyl, carbamylméthyl)aniline, la 3-méthyl 4-amino N,N-(éthyl, carbamylméthyl)aniline, la 4-amino N,N-(éthyl β-pipéridinoéthyl) aniline, la 3-méthyl 4-amino N,N-(éthyl, β-pipéridinoéthyl)aniline, la 4-amino N,N-(éthyl, β-morpholinoéthyl)aniline, la 3-méthyl 4-amino N,N-(éthyl,β-morpholinoéthyl)aniline, la 4-amino N,N-(éthyl,β-acétylaminoéthyl) aniline, la 4-amino N-(β-méthoxyéthyl) aniline, la 3-méthyl 4-amino N,N-(éthyl, β-acétylaminoéthyl) aniline, la 4-amino N,N-(éthyl, β-mésylaminoéthyl) aniline, la 3-méthyl 4-amino N,N-(éthyl, β-mésylaminoéthyl) aniline, la 4-amino N,N-(éthyl, β- sulfoéthyl)aniline, la 3-méthyl 4-amino N,N-(éthyl, β-sulfoéthyl) aniline, la N-[(4'-amino)phényl]-morpholine, la N-[(4'-amino)phényl]pipéridine, la 2-hydroxyéthylparaphénylènediamine, la fluoroparaphénylènediamine, la carboxyparaphénylènediamine, la sulfoparaphénylènediamine, la 2-isopropylparaphénylènediamine, la 2-n-propylparaphénylènediamine, l'hydroxy-2-n-propylparaphénylènediamine, la 2-hydroxyméthylparaphénylènediamine, la N,Ndiméthyl 3-méthyl paraphénylènediamine, la N,N-(éthyl,β-hydroxyéthyl)paraphénylènediamine, la N-(dihydroxypropyl)paraphénylènediamine, la N-4'-aminophénylparaphénylènediamine, la N-phénylparaphénylènediamine.

Ces paraphénylènediamines peuvent être introduites dans la composition tinctoriale, soit sous forme de base libre, soit sous forme de sels, tels que chlorhydrate, bromhydrate ou sulfate.

Parmi les p-aminophénols, on peut citer le p-aminophénol, le 2-méthyl 4-aminophénol, le 3-méthyl 4-aminophénol, le 2-chloro 4-aminophénol, le 3-chloro 4-aminophénol, le 2,6-diméthyl 4-aminophénol, le 3,5-diméthyl, 4-aminophénol, le 2,3-diméthyl 4-aminophénol, le 2,5-diméthyl 4-aminophénol, le 2-hydroxyméthyl 4-aminophénol, le 2-(β-hydroxyéthyl)4-aminophénol, le 2-méthoxy 4-aminophénol, le 3-méthoxy 4-aminophénol, le 3-(β-hydroxyéthoxy)4-aminophénol, le 2-méthoxyméthyl 4-aminophénol, le 2-aminométhyl 4-aminophénol, le 2-β-hydroxyéthylaminométhyl 4-aminophénol, le 2-éthoxyméthyl 4-aminophénol, le 2-(β-hydroxyéthoxy)méthyl 4-aminophénol.

Les bases dites "doubles"sont des bis-phénylalkylènediamines, répondant à la formule : 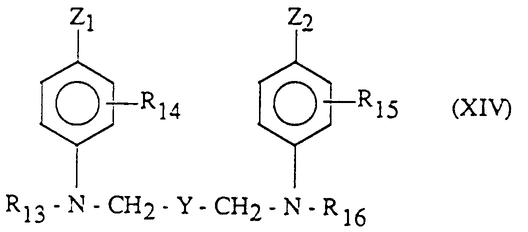
dans laquelle :
Z₁ et Z₂, identiques ou différents, représentent des groupements hydroxyle ou NHR₁₇, où R₁₇ désigne un atome d'hydrogène ou un radical alkyle inférieur ;
R₁₄ et R₁₅, identiques ou différents, représentent soit des atomes d'hydrogène, soit des atomes d'halogène, soit encore des radicaux alkyle ;
R₁₃ et R₁₆, identiques ou différents, représentent un atome d'hydrogène, un radical alkyle, hydroxyalkyle ou aminoalkyle, dont le reste amino peut être substitué; Y représente un radical pris dans le groupe constitué par les radicaux suivants :
-(CH₂)ₙ-, -(CH₂)ₘ-O-(CH₂)ₘ-,
-(CH₂)_{q}-CHOH-(CH₂)_{q}-, 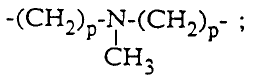
dans lesquels n est un nombre entier compris entre 0 et 8 et m, q et p sont des nombres entiers compris entre 0 et 4, cette base pouvant se présenter également sous forme de ses sels d'addition avec des acides.

Les radicaux alkyle ou alcoxy ci-dessus indiqués désignent de préférence un groupement ayant 1 à 4 atomes de carbone et notamment méthyle, éthyle, propyle, méthoxy et éthoxy.

Parmi les composés de formule (IV) on peut citer le N,N'-bis-(β-hydroxyéthyl)N,N'-bis-(4'-aminophényl) 1,3-diamino 2-propanol, la N,N'-bis-(β-hydroxyéthyl)N,N'-bis-(4'-aminophényl)éthylènediamine, la N,N'-bis-(4-aminophényl)tétraméthylènediamine, la N,N'-bis-(β-hydroxyéthyl) N,N'-bis-(4-aminophényl)tétraméthylènediamine, la N,N'-bis-(4-méthylaminophényl)tétraméthylènediamine, la N,N'-bis(éthyl) N,N'-bis-(4'-amino 3'-méthylphényl)éthylènediamine.

Les précurseurs de colorants d'oxydation de type ortho sont choisis parmi les orthoaminophénols, comme le 1-amino-2-hydroxybenzène, le 6-méthyl 1-hydroxy 2-aminobenzène, le 4-méthyl 1-amino 2-hydroxybenzène et les orthophénylènediamines.

Les compositions définies ci-dessus, appliquées dans la teinture des fibres kératiniques, peuvent également contenir en plus du coupleur répondant à la formule (I) définie ci-dessus, d'autres coupleurs connus en eux-mêmes, tels que des métadiphénols, des métaaminophénols différents de ceux de formule (I), des métaphénylènediamines, des métaacylaminophénols, des métauréidophénols, des métacarbalcoxyaminophénols, l'α-naphtol, des dérivés indoliques, des coupleurs possédant un groupement méthylène actif tels que les composés β-cétoniques, les pyrazolones.

Parmi ces coupleurs, on peut plus particulièrement citer le 2, 4-dihydroxyphénoxyéthanol, le 2,4-dihydroxyanisole, le métaaminophénol, le monométhyléther de résorcine, la résorcine, la 2-méthyl-résorcine, le 2-méthyl 5-aminophénol, le 2-méthyl 5-N-(β-hydroxyéthyl) aminophénol, le 2-méthyl 5-N-(β-mésylaminoéthyl)aminophénol, le 2,6-diméthyl 3-aminophénol, la 6-hydroxybenzomorpholine, le 2,4-diaminoanisole, le 2,4-diaminophénoxyéthanol, la 6-aminobenzomorpholine, le [2-N-(β-hydroxyéthyl) amino 4-amino]-phénoxyéthanol, le 2-amino 4-N-(β-hydroxyéthyl) amino anisole, le (2,4-diamino)phényl-β-γ-dihydroxypropyléther, la 2,4-diaminophénoxyéthylamine, le 1,3-diméthoxy 2,4-diaminobenzène, le 1,3,5-triméthoxy 2,4-diaminobenzène, le 1-amino 3,4-méthylènedioxybenzène, le 1-hydroxy 3,4-méthylènedioxybenzène, le 2-chloro 6-méthyl 3-aminophénol, le 2-méthyl 3-aminophénol, le 2-chloro résorcinol, la 6-méthoxy 3-hydroxyéthylaminoaniline, le 1-éthoxy 2-bis(β-hydroxyéthyl)amino 4-aminobenzène, le 3-diéthylaminophénol, le 1,3-dihydroxy 2-méthylbenzène, le 1-hydroxy 2,4-dichloro 3-aminobenzène, le 4,6-di-(hydroxyéthoxy) 1,3-diaminobenzène, le 4-méthyl 6-éthoxy 1,3-diaminobenzène, le 4-chloro 6-méthyl 3-aminophénol, le 6-chloro 3-trifluoroéthylaminophénol, et leurs sels.

On peut rajouter à ces compositions, comme cela est bien connu dans l'état de la technique, notamment en vue de nuancer ou d'enrichir en reflet les colorations apportées par les précurseurs de colorants d'oxydation, des colorants directs tels que des colorants azoïques, anthraquinoniques ou les dérivés nitrés de la série benzénique.

L'ensemble des précurseurs de colorants par oxydation de type para et/ou ortho, ainsi que les coupleurs utilisés dans les compositions tinctoriales conformes à l'invention, représente de préférence de 0,3 à 7 % en poids par rapport au poids de ladite composition, la concentration en métaaminophénols soufrés de formule (I) peut varier entre 0,05 et 3,5% en poids par rapport au poids de ladite composition.

Les compositions tinctoriales comformes à l'invention, contiennent également dans leur forme de réalisation préférée, des agents tensio-actifs anioniques, cationiques, non-ioniques, amphotères ou leurs mélanges bien connus de l'état de la technique.

Ces agents tensio-actifs sont présents dans les compositions conformes à l'invention dans des proportions comprises entre 0,5 et 55 % en poids, et de préférence entre 2 et 50 % en poids par rapport au poids total de la composition.

Ces compositions peuvent également contenir des solvants organiques pour solubiliser les composants qui ne seraient pas suffisamment solubles dans l'eau. Parmi ces solvants, on peut citer à titre d'exemple, les alcanols inférieurs en C₁-C₄, tels que l'éthanol et l'isopropanol; le glycérol; les glycols ou éthers de glycols comme le 2-butoxyéthanol, l'éthylèneglycol, le propylèneglycol, le monoéthyléther et le monométhyléther du diéthylèneglycol, ainsi que les alcools aromatiques comme l'alcool benzylique ou le phénoxyéthanol, les produits analogues et leurs mélanges.

Les solvants sont présents de préférence dans des proportions comprises entre 1 et 40 % en poids, et en particulier entre 5 et 30 % en poids par rapport au poids total de la composition.

Les agents épaississants que l'on peut ajouter dans les compositions conformes à l'invention peuvent être choisis parmi l'alginate de sodium, la gomme arabique, les polymères d'acide acrylique éventuellement réticulés, les dérivés de cellulose, les hétérobiopolysaccharides tels que la gomme de xanthane. On peut également utiliser des agents épaississants minéraux tels que la bentonite. Ces agents épaississants sont présents de préférence dans des proportions comprises entre 0,1 et 5 %, et en particulier entre 0,2 et 3 % en poids par rapport au poids total de la composition.

Les agents antioxydants qui peuvent être présents dans les compositions sont choisis en particulier parmi le sulfite de sodium, l'acide thioglycolique, le bisulfite de sodium, l'acide déhydroascorbique, l'hydroquinone et l'acide homogentisique. Ces agents antioxydants sont présents dans la composition dans des proportions comprises entre 0,05 et 1,5 % en poids par rapport au poids total de la composition.

Ces compositions peuvent également contenir d'autres adjuvants cosmétiquement acceptables, tels que par exemple des agents de pénétration, des agents séquestrants, des parfums, des tampons, des agents dispersants, des agents de traitement, des agents de conditionnement, des agents filmogènes, des agents conservateurs, des agents opacifiants...

La composition conforme à l'invention contenant un métaaminophénol soufré de formule (I) et un précurseur de colorant d'oxydation de type ortho et/ou para, peut avoir un pH compris entre 3 et 10,5 qui peut être ajusté à la valeur choisie au moyen d'agents alcalinisants habituellement utilisés en teinture des fibres kératiniques, tels que l'ammoniaque, les carbonates alcalins, les alcanolamines tels que les mono-, di- et triéthanolamines, ainsi que leurs dérivés ou des agents acidifiants classiques, tels que les acides minéraux ou organiques, comme les acides chlorhydrique, tartrique, citrique et phosphorique.

La composition appliquée sur les cheveux peut se présenter sous des formes diverses, telles que sous forme de liquides, de crèmes, de gels ou sous toute autre forme appropriée pour réaliser une teinture des fibres kératiniques et notamment des cheveux humains. Ces compositions peuvent être conditionnées sous pression en flacons aérosols en présence d'un agent propulseur et former des mousses.

Les composés de formule (I) sont utilisés conformément à l'invention selon un procédé comprenant l'application sur les fibres kératiniques, et plus particulièrement les cheveux humains, du composé de formule (I) et de précurseurs de colorants d'oxydation de type ortho et/ou para en présence d'un agent oxydant.

L'application de ces composés peut se faire simultanément à l'aide d'une composition tinctoriale telle que définie précédemment ou successivement dans un procédé à plusieurs étapes.

Les compositions tinctoriales conformes à l'invention contenant un précurseur de colorant par oxydation du type para et/ou ortho et un coupleur de formule (I), sont utilisées suivant un procédé mettant en oeuvre la révélation par un agent oxydant.

L'agent oxydant est choisi de préférence parmi le peroxyde d'hydrogène, le peroxyde d'urée, les bromates de métaux alcalins, les persels tels que les perborates et les persulfates. Le peroxyde d'hydrogène est particulièrement préféré.

Conformément à ce procédé on mélange, au moment de l'emploi, la composition tinctoriale décrite ci-dessus avec une solution oxydante en une quantité suffisante pour pouvoir développer une coloration, puis on applique le mélange obtenu sur les fibres kératiniques et en particulier les cheveux humain.

Le pH de la composition appliquée sur les cheveux varie de préférence entre 2 et 13. Il est ajusté à la valeur désirée à l'aide d'agents alcalinisants ou acidifiants bien connus de l'état de la technique et définis précédemment. La solution oxydante contient à titre d'agent oxydant, l'eau oxygénée, le peroxyde d'urée, des persels, tels que le persulfate d'ammonium ou des bromates de métaux alcalins. On utilise de préférence une solution d'eau oxygénée à 20 volumes.

Le mélange obtenu est appliqué sur les cheveux et on laisse poser pendant 10 à 40 minutes, de préférence 15 à 30 minutes, après quoi on les rince, les lave au shampooing, on les rince à nouveau et on les sèche.

Le coupleur de formule (I) définie ci-dessus, peut également être mis en oeuvre dans un procédé à plusieurs étapes, consistant dans l'une des étapes, à appliquer le précurseur de colorant d'oxydation du type ortho et/ou para ou leur mélange et, dans une autre étape, à appliquer une composition tinctoriale contenant le coupleur de formule (I). L'agent oxydant peut être introduit, juste avant l'application, dans la composition appliquée dans le deuxième temps ou bien être appliqué sur les fibres kératiniques elles-mêmes, dans un troisième temps, les conditions de pose, de pH, de lavage et de séchage étant identiques à celles indiquées ci-dessus.

Les exemples qui suivent sont destinés à illustrer l'invention sans pour autant présenter un caractère limitatif.

### EXEMPLE 1

### Préparation du chlorhydrate de 4-méthylthio-3-aminophénol, selon la nomenclature IUPAC chlorhydrate de 3-amino-4-méthyl-sulfanylphénol

### Etape 1 : Synthèse du [2-nitro-4-(benzyloxy)phényl]méthylsulfane.

A une suspension de thiométhylate de sodium (0,35 mole) dans 100 ml de diméthoxyéthane à température ambiante, on ajoute par portions 65,9 g (0,25 mole) de 1-chloro-2-nitro-4-(benzyloxy)benzène.

La réaction est exothermique. On refroidit pour maintenir la température entre 25 et 30°C. Le milieu réactionnel est versé dans un litre d'eau glacée. Le précipité cristallisé est essoré et réempâté dans l'eau.

Après recristallisation de l'éthanol à 96° puis de l'acétate d'isopropyle, on obtient des cristaux orangés (34,4 g) fondant à 78°C et dont l'analyse élémentaire calculée pour C₁₄H₁₃NO₃S est :

| % | C | H | N | O | S |
|---|---|---|---|---|---|
| Calculé | 61,07 | 4,76 | 5,09 | 17,43 | 11,65 |
| Trouvé | 61,30 | 4,81 | 4,90 | 17,34 | 11,57 |

### Etape 2 : Synthèse du 2-méthylsulfanyl-5-(benzyloxy)phénylamine.

On chauffe au reflux de l'alcool un mélange de 2,2 g de chlorure d'ammonium, 15 ml d'eau, 140 ml d'alcool à 96° et 70 g de zinc en poudre fine. On ajoute par portions le [2-nitro-4-(benzyloxy)phényl]méthylsulfane obtenu à l'étape 1 (33,8 g - 0,123 mole), de façon à maintenir le reflux sans chauffage.

La réduction est exothermique. A la fin de l'addition, le chauffage au reflux est prolongé 1/4 d'heure.

Le milieu réactionnel décoloré est filtré bouillant. Par refroidissement du filtrat, on obtient la précipitation de cristaux jaune pâle qui sont essorés et séchés (28,2 g).

Après recristallisation du cyclohexane, ce composé fond à 64°C et son analyse élémentaire calculée pour C₁₄H₁₅NOS est :

| % | C | H | N | O | S |
|---|---|---|---|---|---|
| Calculé | 68,54 | 6,16 | 5,71 | 6,52 | 13,07 |
| Trouvé | 68,40 | 6,11 | 5,61 | 6,72 | 13,11 |

### Etape 3 :

La 2-méthylsulfanyl-5-(benzyloxy)phénylamine (28,2 g - 0,115 mole) obtenue à l'étape 2, est chauffée 1 heure à 90-95°C dans un mélange de 50 ml d'acide chlorhydrique à 36% et 5 ml d'eau.

La suspension est refroidie dans un bain de glace.

Après essorage, séchage sous vide sur potasse et recristallisation de l'éthanol absolu, on obtient des cristaux blancs (8,3 g) de chlorhydrate de 3-amino-4-méthylsulfanylphénol fondant avec décomposirion à 210-214°C et dont l'analyse élémentaire calculée pour C₇H₁₀Cl NOS est :

| % | C | H | N | S | Cl |
|---|---|---|---|---|---|
| Calculé | 43,86 | 5,26 | 7,31 | 16,73 | 18,50 |
| Trouvé | 43,36 | 5,42 | 7,08 | 16,46 | 18,26 |

### EXEMPLE 2

### Préparation du chlorhydrate de 2-méthoxy-4-méthylthio-5-aminophénol, selon la nomenclature IUPAC chlorhydrate de 5-amino-2-méthoxy-4-méthylsulfanylphénol

### Etape 1 : Synthèse du 2-méthoxy-4-méthylsulfanyl-5-nitrophénol.

On chauffe au reflux du méthanol la suspension de 50,5 g (0,237 mole) de méthyl-(6-nitrobenzo[1,3]dioxol-5-yl)-sulfane dans 230 ml de méthanol.

On ajoute goutte à goutte 105 ml d'une solution à 30% de méthylate de sodium dans le méthanol.

Le chauffage au reflux de la suspension est prolongé pendant 4 heures.

On refroidit dans un bain de glace et essore le phénate de sodium du composé attendu. Après dissolution du phénate dans 2 litres d'eau et acidification avec une solution d'acide chlorhydrique concentrée, on essore le précipité cristallisé, réempâte dans l'eau et sèche.

Après recristallisation de l'isopropanol, on obtient 44,5 g de cristaux jaunes qui fondent à 158°C et dont l'analyse élémentaire calculée pour C₈H₉NO₄S est :

| % | C | H | N | O | S |
|---|---|---|---|---|---|
| Calculé | 44,65 | 4,21 | 6,51 | 29,73 | 14,90 |
| Trouvé | 44,62 | 4,21 | 6,42 | 29,95 | 14,72 |

### Etape 2 : réduction

Le composé obtenu à l'étape 1 (18,8 g - 0,0873 mole) est réduit selon le mode opératoire décrit pour l'exemple 1 (étape 3), le milieu réactionnel étant filtré bouillant sur de l'éthanol absolu chlorhydrique.

On obtient 9,6 g de cristaux blancs de chlorhydrate de 5-amino-2-méthoxy-4-méthylsulfanylphénol fondant avec décomposition à 235-240°C et dont l'analyse élémentaire calculée pour C₈H₁₂ClNO₂S est :

| % | C | H | N | O | S | Cl |
|---|---|---|---|---|---|---|
| Calculé | 43,34 | 5,46 | 6,32 | 14,43 | 14,46 | 15,99 |
| Trouvé | 43,55 | 5,52 | 6,20 | 14,67 | 14,22 | 15,80 |

### EXEMPLES DE COMPOSITIONS

### EXEMPLE 1

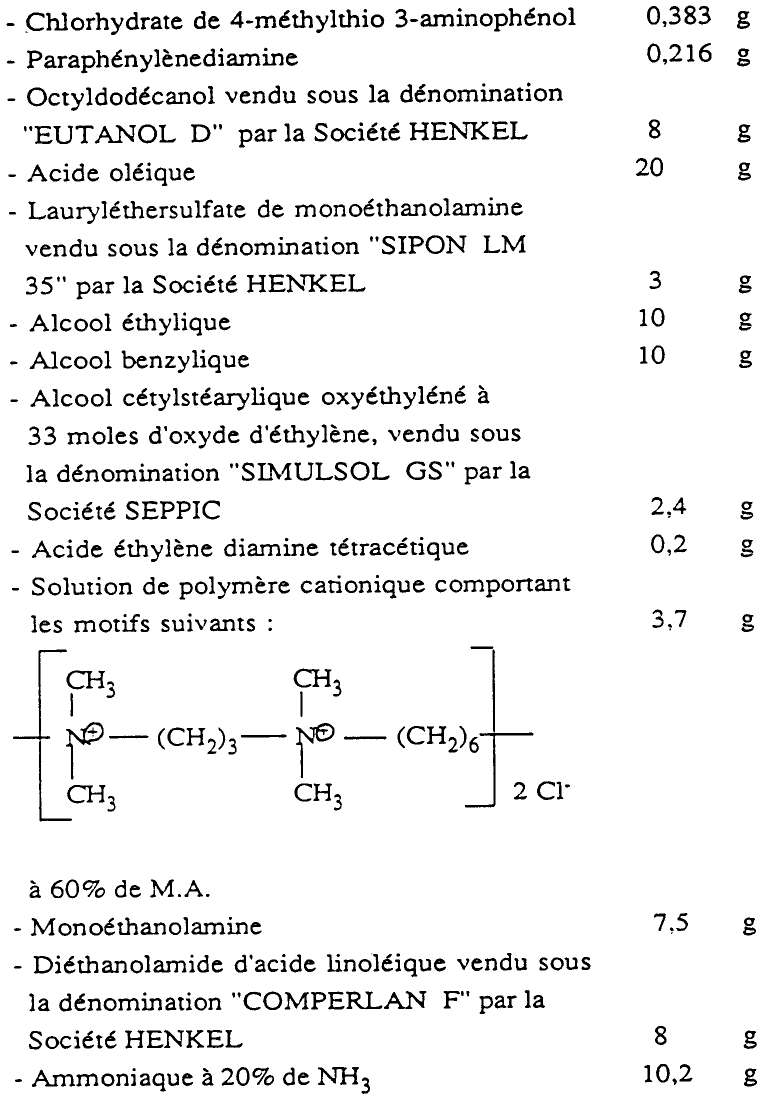

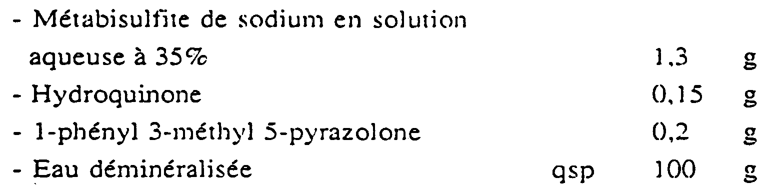

Au moment de l'emploi, on mélange, poids pour poids, la composition ci-dessus à de l'eau oxygénée à 20 volumes dont le pH est égal à 3. Le pH du mélange est égal à 9,5. Celui-ci est appliqué sur des cheveux gris permanentés pendant 30 minutes à température ambiante. Les cheveux sont alors rincés, lavés au shampooing, rincés une nouvelle fois, puis séchés. Ils sont colorés en blond foncé beige.

### EXEMPLE 2

| | |
|---|---|
| - Chlorhydrate de 2-méthoxy 4-méthylthio 5-aminophénol | 0,665 g |
| - Dichlorhydrate de 2,6-diméthylparaphénylènediamine | 0,627 g |
| - Alcool oléique polyglycérolé à 2 moles de glycérol | 4 g |
| - Alcool oléique polyglycérolé à 4 moles de glycérol | 5,7 g |
| - Acide oléique | 3 g |
| - Amine oléique oxyéthylénée à 2 moles d'oxyde d'éthylène, commercialisée sous la dénomination "ETHOMEEN O12" par la Société AKZO | 7 g |
| - Sel de sodium du laurylamino succinamate de diéthylaminopropyle | 3 g |
| - Alcool oléique | 5 g |
| - Diéthanolamide d'acide oléique | 12 g |
| - Propylèneglycol | 3,5 g |
| - Alcool éthylique | 7 g |
| - Dipropylèneglycol | 0,5 g |
| - Monométhyléther de propylèneglycol | 9 g |
| - Métabisulfite de sodium en solution aqueuse à 35% | 0,45 g MA |
| - Acétate d'ammonium | 0,8 g |
| - Antioxydant, séquestrant qs | |
| - Parfum, conservateur qs | |
| - Monoéthanolamine qs pH=9,8 | |
| - Eau déminéralisée qsp | 100 g |

Au moment de l'emploi, on mélange la composition ci-dessus, à un poids égal d'eau oxygénée à 20 volumes dont le pH est ajusté entre 1 et 1,5 par addition d'acide orthophosphorique (2,5 g d'acide orthophosphorique pour 100 g d'eau oxygénée à 20 volumes).

Le pH du mélange est égal à 6,5. Celui-ci est appliqué sur des cheveux gris à 90% de blancs pendant 30 minutes à température ambiante. Les cheveux sont ensuite rincés, lavés au shampooing, rincés une nouvelle fois puis séchés. Ils sont colorés en blond clair cendré bleuté.

## Revendications

1. Métaaminophénols soufrés de formule générale : 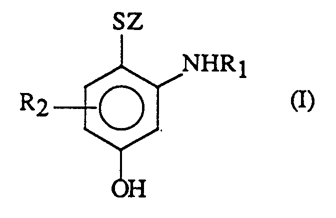 dans laquelle Z représente un radical alkyle en C₁-C₁₈, un radical aralkyle dans lequel le radical alkyle est en C₁-C₆, un radical monohydroxyalkyle en C₁-C₆ ou polyhydroxyalkyle en C₂-C₆, un radical aryle, un radical aminoalkyle de formule : 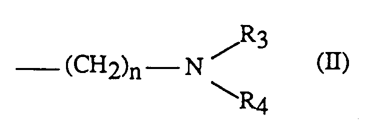 dans laquelle :
n est un nombre entier compris entre 1 et 6 inclus;
R₃ et R₄, identiques ou différents, représentent un atome d'hydrogène ou un radical alkyle en C₁-C₄, hydroxyalkyle en C₁-C₄, acyle en C₁-C₆;
R₁ représente un atome d'hydrogène, un radical alkyle en C₁-C₆, un radical monohydroxyalkyle en C₁-C₆, polyhydroxyalkyle en C₂-C₆, monocarbamylalkyle en C₁-C₆, dicarbamylalkyle en C₁-C₆, aminoalkyle en C₁-C₆, acyl(C₁-C₆)aminoalkyle(C₁-C₄), carbalcoxy (C₂-C₆)alkyle(C₁-C₄), carbamyle ou monoalkyl en C₁-C₆ carbamyle;
R₂ représente un atome d'hydrogène, un radical alkyle en C₁-C₄, un radical monohydroxyalkyle en C₁-C₄, un radical alcoxy en C₁-C₄,
ainsi que leurs sels d'acides.

2. Métaaminophénols soufrés selon la revendication 1, caractérisés en ce que Z désigne un radical méthyle, éthyle, propyle, butyle, dodécyle, hexadécyle, benzyle, phényle, ou un radical mono-ou polyhydroxyalkyle choisi parmi :
-CH₂-CH₂-OH, -CH₂-CHOH-CH₂-OH, -CH₂-CHOH-CH₃ ,
ou un radical aminoalkyle choisi parmi :
-CH₂-CH₂NH₂ , -CH₂-CH₂-NHCH₃ ,
ou un radical acylaminoalkyle choisi parmi :
-CH₂-CH₂-NH-COCH₃ ou 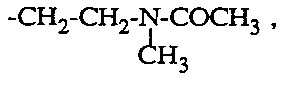 R₂ désigne un atome d'hydrogène ou un radical alcoxy en C₁-C₄.

3. Métaaminophénols soufrés selon la revendication 1 ou 2, caractérisée par le fait que les sels d'acides correspondants sont choisis parmi les chlorhydrates, les sulfates, ou les bromhydrates.

4. Métaaminophénols soufrés selon l'une quelconque des revendications 1 à 3, caractérisés en ce qu'il s'agit des composés suivants :
le 2-méthoxy 4-méthylthio 5-aminophénol
le 4-méthylthio 3-aminophénol
ou, selon la nomenclature IUPAC :
le 5-amino 2-méthoxy 4-méthylsulfanylphénol
le 3-amino 4-méthylsulfanylphénol.

5. Nitrobenzènes soufrés de formule générale : 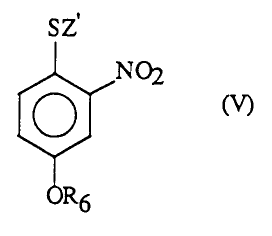 dans laquelle Z' représente un radical alkyle en C₁-C₁₈, un radical aralkyle dans lequel le radical alkyle est en C₁-C₆, un radical monohydroxyalkyle en C₁-C₆ ou polyhydroxyalkyle en C₂-C₆, un radical aryle, un radical aminoalkyle de formule : 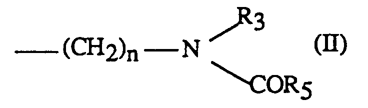 dans laquelle :
n est un nombre entier compris entre 1 et 6 inclus;
R₃ représente un atome d'hydrogène ou un radical alkyle en C₁-C₄, hydroxyalkyle en C₁-C₄, acyle en C₂-C₆;
R₅ représente un atome d'hydrogène ou un radical alkyle en C₁-C₃;
R₆ représente un atome d'hydrogène, un radical alkyle en C₁-C₁₈, un radical aralkyle, un radical silyle, un radical pyranyle, en particulier pour la préparation des métaaminophénols soufrés de formule (I) selon la revendication 1, sous réserve que :
. lorsque R₆ désigne l'atome d'hydrogène, Z ne peut représenter un radical alkyle en C₁-C₄ ou en C₁₂ ou un radical aryle, et
. lorsque R₆ désigne le radical méthyle, Z ne peut représenter le radical méthyle et benzyle.

6. Nitrobenzènes soufrés selon la revendication 5, caractérisés en ce qu'il s'agit des composés suivants :
le 4-méthylthio 3-nitrophénol
le 2-méthylthio 5-benzyloxynitrobenzène.

7. Composés de formule générale : 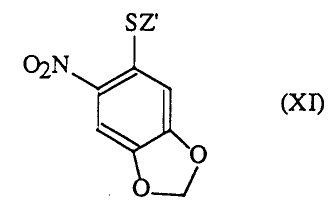 dans laquelle Z' représente un radical alkyle en C₅-C₁₈, un radical aralkyle dans lequel le radical alkyle est en C₂-C₆, un radical mono-hydroxyalkyle en C₁-C₆ ou polyhydroxyalkyle en C₂-C₆, un radical aminoalkyle de formule : 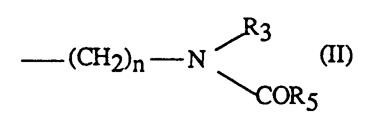 dans laquelle :
n est un nombre entier compris entre 1 et 6 inclus;
R₃ un atome d'hydrogène ou un radical alkyle en C₁-C₄, hydroxyalkyle en C₁-C₄, acyle en C₁-C₆;
R₅ représente un atome d'hydrogène ou un radical alkyle en C₁-C₃, en particulier pour la préparation des métaaminophénols soufrés de formule (I) selon la revendication 1.

8. Composés nouveaux répondant à la formule : 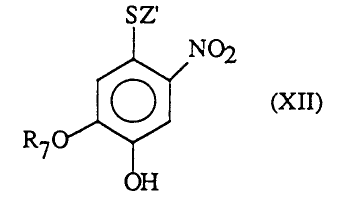 dans laquelle :
Z' représente un radical alkyle en C₁-C₁₈, un radical aralkyle dans lequel le radical alkyle est en C₁-C₆, un radical monohydroxyalkyle en C₁-C₆ ou polyhydroxyalkyle en C₂-C₆, un radical aryle, un radical aminoalkyle de formule : 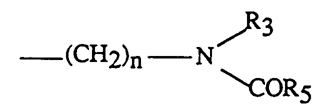 dans laquelle :
n est un nombre entier compris entre 1 et 6 inclus;
R₃ un atome d'hydrogène ou un radical alkyle en C₁-C₄, hydroxyalkyle en C₁-C₄, acyle en C₁-C₆;
R₅ représente un atome d'hydrogène ou un radical alkyle en C₁-C₃;
R₇ représente un radical alkyle en C₁-C₄ linéaire ou ramifié, en particulier pour la préparation des métaaminophénols soufrés de formule (I) selon la revendication 1.

9. Composé selon la revendication 8, caractérisé en ce qu'il s'agit du 2-méthoxy 4-méthylthio 5-nitrophénol.

10. Utilisation en teinture des fibres kétatiniques et, plus particulièrement des cheveux humains, de métaaminophénols soufrés de formule générale : 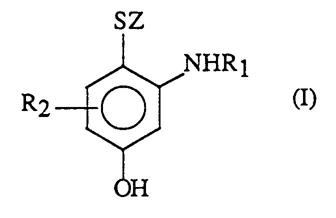 dans laquelle Z représente un radical alkyle en C₁-C₁₈, un radical aralkyle dans lequel le radical alkyle est en C₁-C₆, un radical monohydroxyalkyle en C₁-C₆ ou polyhydroxyalkyle en C₂-C₆, un radical aryle, un radical aminoalkyle de formule : 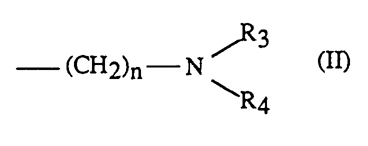 dans laquelle :
n est un nombre entier compris entre 1 et 6 inclus;
R₃ et R₄, identiques ou différents, représentent un atome d'hydrogène ou un radical alkyle en C₁-C₄, hydroxyalkyle en C₁-C₄, acyle en C₁-C₆;
R₁ représente un atome d'hydrogène, un radical alkyle en C₁-C₆, un radical monohydroxyalkyle en C₁-C₆, polyhydroxyalkyle en C₂-C₆, monocarbamylalkyle en C₁-C₆, dicarbamylalkyle en C₁-C₆, aminoalkyle en C₁-C₆, acyl(C₁-C₆)aminoalkyle(C₁-C₄), carbalcoxy (C₂-C₆)alkyle(C₁-C₄), carbamyle ou monoalkyl en C₁-C₆ carbamyle;
R₂ représente un atome d'hydrogène, un radical alkyle en C₁-C₄, un radical monohydroxyalkyle en C₁-C₄, un radical alcoxy en C₁-C₄.

11. Composition tinctoriale pour fibres kératiniques, et plus particulièrement les cheveux humains, caractérisée en ce qu'elle contient dans un milieu approprié pour la teinture, au moins un précurseur de colorant d'oxydation de type ortho et/ou para et au moins, à titre de coupleur, un métaaminophénol soufré de formule : 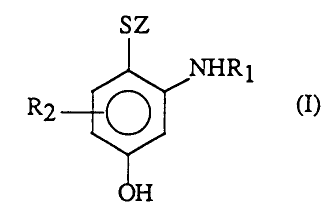 dans laquelle Z représente un radical alkyle en C₁-C₁₈, un radical aralkyle dans lequel le radical alkyle est en C₁-C₆, un radical monohydroxyalkyle en C₁-C₆ ou polyhydroxyalkyle en C₂-C₆, un radical aryle, un radical aminoalkyle de formule : 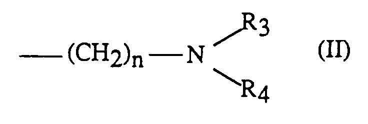 dans laquelle :
n est un nombre entier compris entre 1 et 6 inclus;
R₃ et R₄, identiques ou différents, représentent un atome d'hydrogène ou un radical alkyle en C₁-C₄, hydroxyalkyle en C₁-C₄, acyle en C₁-C₆;
R₁ représente un atome d'hydrogène, un radical alkyle en C₁-C₆, un radical monohydroxyalkyle en C₁-C₆, polyhydroxyalkyle en C₂-C₆, monocarbamylalkyle en C₁-C₆, dicarbamylalkyle en C₁-C₆, aminoalkyle en C₁-C₆, acyl(C₁-C₆)aminoalkyle(C₁-C₄), carbalcoxy (C₂-C₆)alkyle(C₁-C₄), carbamyle ou monoalkyl en C₁-C₆ carbamyle;
R₂ représente un atome d'hydrogène, un radical alkyle en C₁-C₄, un radical monohydroxyalkyle en C₁-C₄, un radical alcoxy en C₁-C₄,
et leurs sels correspondants.

12. Composition tinctoriale selon la revendication 11, caractérisée par le fait que les précurseurs de colorants d'oxydation de type ortho ou para sont choisis parmi les paraphénylènediamines, les paraaminophénols, les précurseurs hétérocycliques para dérivés de la pyridine, de la pyrimidine ou du pyrazole, les orthoaminophénols et les bisphénylalkylènediamines.

13. Composition tinctoriale selon l'une des revendications 11 ou 12, caractérisée en ce que les composés de formule (I) sont choisis parmi les composés suivants :
le 2-méthoxy 4-méthylthio 5-aminophénol
le 4-méthylthio 3-aminophénol,
ou leurs sels d'addition avec un acide.

14. Composition tinctoriale selon l'une quelconque des revendications 11 à 13, caractérisée en ce qu'elle contient outre les coupleurs de formule (I), d'autres coupleurs tels que des métadiphénols, des métaphénylènediamines, des métaaminophénols différents de ceux de formule (I), des métaacylaminophénols, des métauréidophénols, des métacarbalcoxyaminophénols, l'α-naphtol, des dérivés indoliques, des coupleurs possédant un groupe méthylène actif.

15. Composition tinctoriale selon l'une quelconque des revendications 11 à 14, caractérisée en ce qu'elle contient 0,05 à 3,5% en poids du poids total de la composition d'au moins un composé de formule (I).

16. Composition tinctoriale selon l'une quelconque des revendications 11 à 15, caractérisée en ce qu'elle contient de 0,3 à 7% en poids par rapport au poids total de la composition de précurseurs de colorant d'oxydation de type ortho et/ou para et de coupleurs.

17. Composition tinctoriale selon l'une quelconque des revendications 11 à 16, caractérisée en ce qu'elle contient en outre un composé choisi parmi les agents tensio-actifs cationiques, anioniques, non-ioniques, amphotères ou leurs mélanges, en des concentrations comprises entre 0,5% et 55% en poids par rapport au poids total de la composition; les solvants organiques en des concentrations comprises entre 1 et 40% en poids par rapport au poids total de la composition; les agents épaississants en des concentrations comprises entre 0,1 et 5% en poids par rapport au poids total de la composition; les agents antioxydants en des proportions comprises entre 0,05 et 1,5% en poids par rapport au poids total de la composition et les colorants directs.

18. Composition tinctoriale selon l'une quelconque des revendications 11 à 17, caractérisée en ce qu'elle a un pH compris entre 3 et 10,5.

19. Procédé de teinture d'oxydation des fibres kératiniques et en particulier des cheveux humains, caractérisé en ce que l'on applique sur lesdites fibres un ou plusieurs précurseurs de colorants d'oxydation de type ortho et/ou para et un métaaminophénol soufré de formule générale : 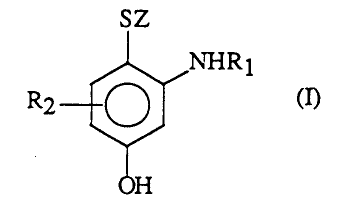 dans laquelle Z représente un radical alkyle en C₁-C₁₈, un radical aralkyle dans lequel le radical alkyle est en C₁-C₆, un radical monohydroxyalkyle en C₁-C₆ ou polyhydroxyalkyle en C₂-C₆, un radical aryle, un radical aminoalkyle de formule : 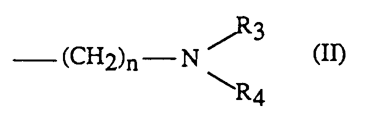 dans laquelle :
n est un nombre entier compns entre 1 et 6 inclus;
R₃ et R₄, identiques ou différents, représentent un atome d'hydrogène ou un radical alkyle en C₁-C₄, hydroxyalkyle en C₁-C₄, acyle en C₁-C₆;
R₁ désigne un atome d'hydrogène, un radical alkyle en C₁-C₆, un radical monohydroxyalkyle en C₁-C₆, polyhydroxyalkyle en C₂-C₆, monocarbamylalkyle en C₁-C₆, dicarbamylalkyle en C₁-C₆, aminoalkyle en C₁-C₆, acyl(C₁-C₆)aminoalkyle(C₁-C₄), carbalcoxy (C₂-C₆)alkyle(C₁-C₄), carbamyle ou monoalkyl en C₁-C₆ carbamyle;
R₂ représente un atome d'hydrogène, un radical alkyle en C₁-C₄, un radical monohydroxyalkyle en C₁-C₄, un radical alcoxy en C₁-C₄,
et leurs sels d'acides, en présence d'un agent oxydant.

20. Procédé de teinture selon la revendication 19, caractérisé en ce que l'on mélange, au moment de l'emploi, une composition tinctoriale pour fibres kératiniques et en particulier les cheveux humains, comprenant, dans un milieu approprié pour la teinture, au moins un précurseur de colorant d'oxydation de type ortho et/ou para, et au moins, à titre de coupleur, un métaaminophénol soufré de formule (I), avec une solution oxydante en une quantité suffisante pour développer la coloration, puis on applique le mélange obtenu sur les fibres kératiniques et en particulier les cheveux humains, le pH du mélange étant compris entre 2 et 13, qu'on laisse poser pendant 10 à 40 minutes, de préférence 15 à 30 minutes, on rince les cheveux, on les lave au shampooing, on les rince à nouveau et on les sèche.

## Claims

1. Sulphated metaaminophenols having general formula: 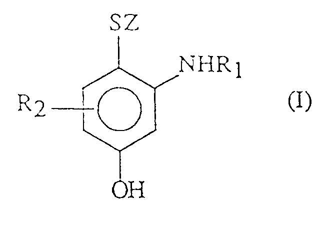 wherein Z represents a C₁-C₁₈ alkyl radical, an aralkyl radical wherein the alkyl radical is C₁-C₆, a C₁-C₆ monohydroxyalkyl or C₂-C₆ polyhydroxyalkyl radical, an aryl radical, an aminoalkyl radical having the formula: 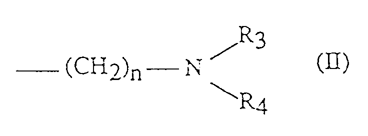 wherein:
n is a whole number from 1 to 6 inclusive;
R₃ and R₄, which may be identical or different, represent a hydrogen atom or a C₁-C₄ alkyl radical, a C₁-C₄ hydroxyalkyl radical or a C₁-C₆ acyl radical;
R₁ represents a hydrogen atom, a C₁-C₆ alkyl radical, a C₁-C₆ monohydroxyalkyl radical, a C₂-C₆ polyhydroxyalkyl radical, a C₁-C₆ monocarbamylalkyl radical, a C₁-C₆ dicarbamylalkyl radical, a C₁-C₆ aminoalkyl radical, an acyl(C₁-C₆)aminoalkyl(C₁-C₄) radical, a carbalkoxy(C₂-C₆)alkyl(C₁-C₄) radical, a carbamyl or monoalkyl C₁-C₆ carbamyl radical;
R₂ represents a hydrogen atom, a C₁-C₄ alkyl radical, a C₁-C₄ monohydroxyalkyl radical, or a C₁-C₄ alkoxy radical,
and their acid salts.

2. Sulphated metaaminophenols according to claim 1 characterised in that Z designates methyl, ethyl, propyl, butyl, dodecyl, hexadecyl, benzyl, phenyl or a mono or polyhydroxyalkyl radical selected from:
-CH₂-CH₂-OH, -CH₂-CHOH-CH₂-OH, -CH₂-CHOH-CH₃;
or an aminoalkyl radical selected from:
-CH₂-CH₂-NH₂, -CH₂-CH₂-NHCH₃;
or an acylaminoalkyl radical selected from:
-CH₂-CH₂-NH-COCH₃; or 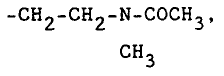 and R₂ designates a hydrogen atom or a C₁-C₄ alkoxy radical.

3. Sulphated metaaminophenols according to claim 1 or claim 2 characterised in that the corresponding acid salts are selected from hydrochlorides, sulphates or hydrobromides.

4. Sulphated metaaminophenols according to any one of claims 1 to 3 characterised in that they are in the form of:
2-methoxy 4-methylthio 5-aminophenol
4-methylthio 3-aminophenol
or, in accordance with IUPAC nomenclature:
5-amino 2-methoxy 4-methylsulphanylphenol
3-amino 4-methylsulphanylphenol.

5. Sulphated nitrobenzenes having general formula: 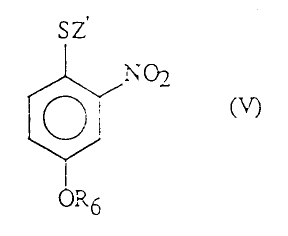 wherein Z' represents a C₁-C₁₈ alkyl radical, an aralkyl radical wherein the alkyl radical is C₁-C₆, a C₁-C₆ monohydroxyalkyl radical, a C₂-C₆ polyhydroxyalkyl radical, an aryl radical, an aminoalkyl radical having formula: 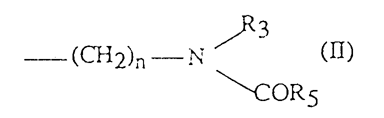 wherein:
n is a whole number between 1 and 6 inclusive;
R₃ represents a hydrogen atom or a C₁-C₄ alkyl radical, a C₁-C₄ hydroxyalkyl radical or a C₁-C₆ acyl radical;
R₅ represents a hydrogen atom or a C₁-C₃ alkyl radical;
R₆ represents a hydrogen atom, a C₁-C₁₈ alkyl radical, an aralkyl radical, a silyl radical or a pyranyl radical,
particularly for preparation of sulphated metaaminophenols having formula (I), providing that:
. when R₆ designates a hydrogen atom Z does not represent a C₁-C₄ or C₁₂ alkyl radical or an aryl radical, and
. when R₆ designates a methyl radical Z does not represent a methyl or benzyl radical.

6. Sulphated nitrobenzenes according to claim 5 characterised in that they are in the form of:
4-methylthio 3-nitrophenol
2-methylthio 5-benzyloxynitrobenzene.

7. Compound having general formula: 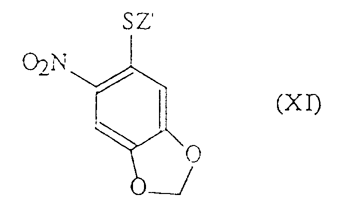 wherein Z' represents a C₅-C₁₈ alkyl radical, an aralkyl radical wherein the alkyl radical is C₂-C₆, a C₁-C₆ monohydroxyalkyl radical, a C₂-C₆ polyhydroxyalkyl radical, an aminoalkyl radical having formula: 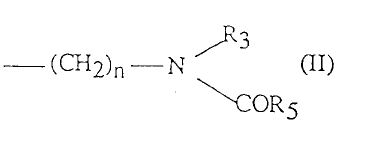 wherein:
n is a whole number between 1 and 6 inclusive;
R₃ represents a hydrogen atom, a C₁-C₄ alkyl radical, a C₁-C₄ hydroxyalkyl radical or a C₁-C₆ acyl radical;
R₅ represents a hydrogen atom or a C₁-C₃ alkyl radical,
particularly for the preparation of sulphated metaaminophenols having formula (I) according to claim 1.

8. Novel compounds having the formula: 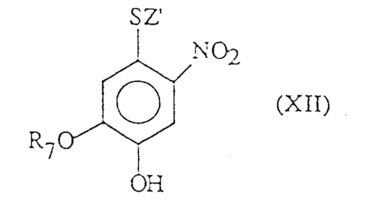 wherein:
Z' represents a C₁-C₁₈ alkyl radical, an aralkyl radical wherein the alkyl radical is C₁-C₆, a C₁-C₆ monohydroxyalkyl radical, a C₂-C₆ polyhydroxyalkyl radical, an aryl radical, an aminoalkyl radical having formula: 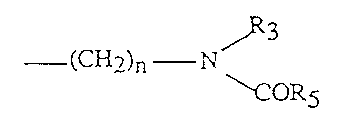 wherein:
n is a whole number between 1 and 6 inclusive;
R₃ represents a hydrogen atom, a C₁-C₄ alkyl radical, a C₁-C₄ hydroxyalkyl radical or a C₁-C₆ acyl radical;
R₅ represents a hydrogen atom or a C₁-C₃ alkyl radical;
R₇ represents a linear or branched C₁-C₄ alkyl radical,
particularly for the preparation of sulphated metaaminophenols having formula (I) in accordance with claim 1.

9. Compound according to claim 8 characterised in that it is in the form of 2-methoxy 4-methylthio 5-nitrophenol.

10. Use in dyeing keratinous fibres, in particular human air, of sulphated metaaminophenols having general formula: 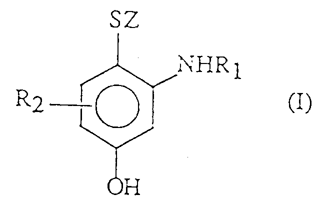 wherein Z represents a C₁-C₁₈ alkyl radical, an aralkyl radical wherein the alkyl radical is C₁-C₆, a C₁-C₆ monohydroxyalkyl or C₂-C₆ polyhydroxyalkyl radical, an aryl radical, an aminoalkyl radical having the formula: 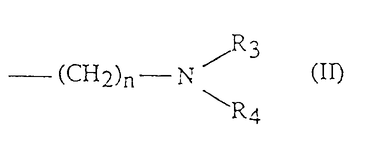 wherein:
n is a whole number from 1 to 6 inclusive;
R₃ and R₄, which may be identical or different, represent a hydrogen atom, a C₁-C₄ alkyl radical, a C₁-C₄ hydroxyalkyl radical or a C₁-C₆ acyl radical;
R₁ represents a hydrogen atom, a C₁-C₆ alkyl radical, a C₁-C₆ monohydroxyalkyl radical, a C₂-C₆ polyhydroxyalkyl radical, a C₁-C₆ monocarbamylalkyl radical, a C₁-C₆ dicarbamylalkyl radical, a C₁-C₆ aminoalkyl radical, an acyl(C₁-C₆)aminoalkyl(C₁-C₄) radical, a carbalkoxy(C₂-C₆)alkyl(C₁-C₄) radical, a carbamyl or monoalkyl C₁-C₆ carbamyl radical;
R₂ represents a hydrogen atom, a C₁-C₄ alkyl radical, a C₁-C₄ monohydroxyalkyl radical or a C₁-C₄ alkoxy radical.

11. A dye composition for keratinous fibres, in particular human hair, characterised in that it contains in an appropriate dye medium at least one ortho and/or para type oxidation dye precursor and at least one sulphated metaaminophenol acting as coupling agent and having the formula: 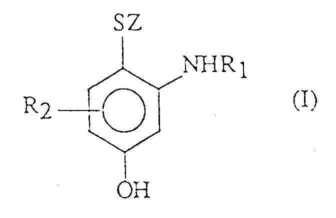 wherein Z represents a C₁-C₁₈ alkyl radical, an aralkyl radical wherein the alkyl radical is C₁-C₆, a C₁-C₆ monohydroxyalkyl or C₂-C₆ polyhydroxyalkyl radical, an aryl radical, an aminoalkyl radical having the formula: 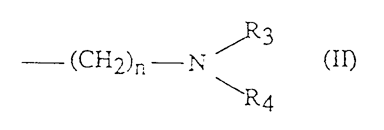 wherein:
n is a whole number from 1 to 6 inclusive;
R₃ and R₄, which may be identical or different, represent a hydrogen atom or a C₁-C₄ alkyl radical, a C₁-C₄ hydroxyalkyl radical or a C₁-C₆ acyl radical;
R₁ represents a hydrogen atom, a C₁-C₆ alkyl radical, a C₁-C₆ monohydroxyalkyl radical, a C₂-C₆ polyhydroxyalkyl radical, a C₁-C₆ monocarbamylalkyl radical, a C₁-C₆ dicarbamylalkyl radical, a C₁-C₆ aminoalkyl radical, an acyl(C₁-C₆)aminoalkyl(C₁-C₄) radical, a carbalkoxy(C₂-C₆)alkyl(C₁-C₄) radical, a carbamyl or monoalkyl C₁-C₆ carbamyl radical;
R₂ represents a hydrogen atom, a C₁-C₄ alkyl radical, a C₁-C₄ monohydroxyalkyl radical or a C₁-C₄ alkoxy radical,
and their corresponding salts.

12. Dye composition according to claim 11 characterised in that the ortho or para type oxidation dye precursors are selected from paraphenylenediamines, paraaminophenols, para heterocyclic derivatives of pyridine, pyrimidine or pyrazole, orthoaminophenols and bis-phenylalkylenediamines.

13. Dye composition according to claim 11 or claim 12 characterised in that the compounds having formula (I) are selected from the following:
2-methoxy 4-methylthio 5-aminophenol,
4-methylthio 3-aminophenol,
or their acid addition salts.

14. Dye composition according to any one of claims 11 to 13 characterised in that it further contains coupling agents having formula (I) and other coupling agents such as metadiphenols, metaphenylenediamines, metaaminophenols other than those having formula (I), metaacylaminophenols, metaureidophenols, metacarbalkoxyaminophenols, α-napthol, indole derivatives and coupling agents having an active methylene group.

15. Dye composition according to any one of claims 11 to 14 characterised in that it contains 0.05 to 3.5% by weight with respect to the total composition weight of at least one compound having formula (I).

16. Dye composition according to any one of claims 11 to 15 characterised in that it contains 0.3 to 7% by weight with respect to the total composition weight of ortho and/or para type oxidation dye precursors and coupling agents.

17. Dye composition according to any one of claims 11 to 16 characterised in that it further contains a compound selected from a cationic, anionic, nonionic or amphoteric surfactant or mixture thereof in a concentration of between 0.5 and 55% by weight with respect to the total composition weight, organic solvents in a concentration of between 1 and 40% by weight with respect to the total composition weight, thickening agents in a concentration of between 0.1 and 5% by weight with respect to the total composition weight, antioxidants in a proportion of between 0.05 and 1.5% by weight with respect to the total composition weight and a direct dye.

18. Dye composition according to any one of claims 11 to 17 characterised in that the pH is between 3 and 10.5.

19. A method of dyeing keratinous fibres, in particular human hair, characterised in that one or several ortho and/or oxidation dye precursors is applied to said fibres along with a sulphated metaaminophenol having general formula: 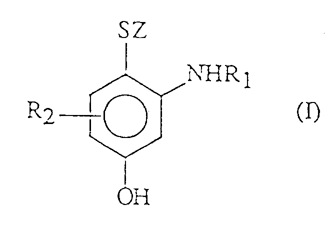 wherein Z represents a C₁-C₁₈ alkyl radical, an aralkyl radical wherein the alkyl radical is C₁-C₆, a C₁-C₆ monohydroxyalkyl or C₂-C₆ polyhydroxyalkyl radical, an aryl radical, an aminoalkyl radical having the formula: 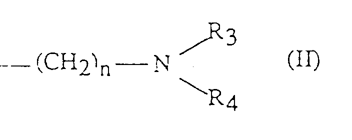 wherein:
n is a whole number from 1 to 6 inclusive;
R₃ and R₄, which may be identical or different, represent a hydrogen atom, a C₁-C₄ alkyl radical, a C₁-C₄ hydroxyalkyl radical or a C₁-C₆ acyl radical;
R₁ represents a hydrogen atom, a C₁-C₆ alkyl radical, a C₁-C₆ monohydroxyalkyl radical, a C₂-C₆ polyhydroxyalkyl radical, a C₁-C₆ monocarbamylalkyl radical, a C₁-C₆ dicarbamylalkyl radical, a C₁-C₆ aminoalkyl radical, an acyl(C₁-C₆)aminoalkyl(C₁-C₄) radical, a carbalkoxy(C₂-C₆)alkyl(C₁-C₄) radical, a carbamyl or monoalkyl C₁-C₆ carbamyl radical;
R₂ represents a hydrogen atom, a C₁-C₄ alkyl radical, a C₁-C₄ monohydroxyalkyl radical or a C₁-C₄ alkoxy radical,
and their acid salts, in the presence of an oxidising agent.

20. Dyeing method according to claim 19 characterised in that a dye composition for keratinous fibres, particularly human hair, comprising at least one ortho and/or para type oxidation dye precursor and at least one sulphated metaaminophenol having formula (I) as coupling agent in an appropriate dye medium is mixed just before use with a sufficient amount of an oxidising solution to develop the dye, the mixture obtained being then applied to the keratinous fibres, in particular human hair, the pH of the mixture being between 2 and 13, and left on the hair for 10 to 40 minutes, preferably 15 to 30 minutes, the hair being then rinsed, shampooed, rinsed again and dried.

## Patentansprüche

1. Schwefelhaltige m-Aminophenole der allgemeinen Formel : 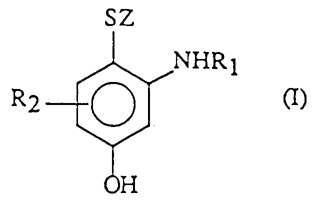 worin Z einen C₁₋₁₈-Alkylrest, einen Aralkylrest, worin der Alkylrest ein C₁₋₆-Rest ist, einen C₁₋₆-Monohydroxyalkyl- oder C₂₋₆-Polyhydroxyalkylrest, einen Arylrest und einen Aminoalkylrest der Formel darstellt: 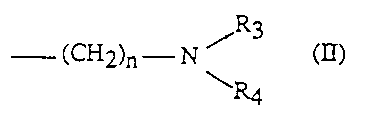 worin gilt:
n ist eine ganze Zahl von 1 bis 6;
R₃ und R₄ stellen, gleich oder verschieden, ein Wasserstoffatom oder einen C₁₋₄-Alkyl-, C₁₋₄-Hydroxyalkyl- oder C₁₋₆-Acylrest dar;
und worin R₁ ein Wasserstoffatom, einen C₁₋₆-Alkyl-, C₁₋₆-Monohydroxyalkyl-, C₂₋₆-Polyhydroxyalkyl-, C₁₋₆-Monocarbamylalkyl-, C₁₋₆-Dicarbamylalkyl-, C₁₋₆-Aminoalkyl-, C₁₋₆-Acylamino-C₁₋₄-alkyl-, C₂₋₆-Carbalkoxy-C₁₋₄-alkyl-, Carbamyl- oder einen C₁₋₆-Monoalkylcarbamylrest und
R₂ ein Wasserstoffatom, einen C₁₋₄-Alkyl-, C₁₋₄-Monohydroxyalkyl- oder einen C₁₋₄-Alkoxyrest darstellen,
sowie deren Salze mit Säuren.

2. Schwefelhaltige m-Aminophenole gemäß Anspruch 1,
dadurch **gekennzeichnet**, daß
Z einen Methyl-, Ethyl-, Propyl-, Butyl-, Dodecyl-, Hexadecyl-, Benzyl-, Phenyl- oder einen Mono- oder Polyhydroxyalkylrest, ausgewählt aus:
-CH₂-CH₂-OH, -CH₂-CHOH-CH₂-OH, -CH₂-CHOH-CH₃,
oder einen Aminoalkylrest, ausgewählt aus:
-CH₂-CH₂-NH₂, -CH₂-CH₂-NHCH₃,
oder einen Acylaminoalkylrest, ausgewählt aus:
-CH₂-CH₂-NH-COCH₃ oder -CH₂-CH₂ -NCH₃-COCH₃,
und R₂ ein Wasserstoffatom oder eine C1-4-Alkoxyrest bedeuten.

3. Schwefelhaltige m-Aminophenole gemäß Anspruch 1 oder 2,
dadurch **gekennzeichnet**, daß
die entsprechenden Säuresalze aus Hydrochloriden, Sulfaten oder Hydrobromiden ausgewählt sind.

4. Schwefelhaltige m-Aminophenole nach einem der Ansprüche
1 bis 3,
dadurch **gekennzeichnet**, daß
es sich um die folgenden Verbindungen handelt:
2-Methoxy-4-methylthio-5-aminophenol,
4-Methylthio-3-aminophenol,
oder gemäß IUPAC-Nomenklatur:
5-Amino-2-methoxy-4-methylsulfanylphenol,
3-Amino-4-methylsulfanylphenol.

5. Schwefelhaltige Nitrobenzole der allgemeinen Formel: 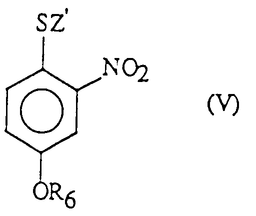 worin Z' einen C₁₋₁₈-Alkylrest, einen Aralkylrest, worin der Alkylrest ein C₁₋₆-Rest ist, einen C₁₋₆-Monohydroxyalkyl- oder C₂₋₆-Polyhydroxyalkylrest, einen Arylrest oder einen Aminoalkylrest der Formel darstellt: 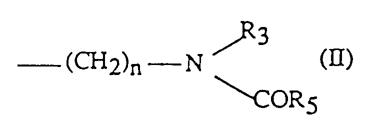 worin gilt:
n ist eine ganze Zahl von 1 bis 6;
R₃ stellt ein Wasserstoffatom oder einen C₁₋₄-Alkylrest, C₁₋₄-Hydroxyalkyl- oder C₂₋₆-Acylrest dar;
R₅ stellt ein Wasserstoffatom oder einen C₁₋₃-Alkylrest dar;
und worin R₆ ein Wasserstoffatom, einen C₁₋₁₈-Alkyl-, Aralkyl-, Silyl- oder Pyranylrest darstellt,
insbesondere zur Herstellung der schwefelhaltigen m-Aminophenole der Formel (I) gemäß Anspruch 1, mit der Maßgabe, daß:
- wenn R₆ ein Wasserstoffatom bedeutet, Z keinen C₁₋₄-Alkyl- oder C₁₂-oder Arylrest darstellt, und
- wenn R₆ den Methylrest bedeutet, Z keinen Methyl- und Benzylrest darstellt.

6. Schwefelhaltige Nitrobenzole gemäß Anspruch 5,
dadurch **gekennzeichnet**, daß
es sich um die folgenden Verbindungen handelt:
das 4-Methylthio-3-nitrophenol,
das 2-Methylthio-5-benzyloxynitrobenzol.

7. Verbindungen der allgemeinen Formel: 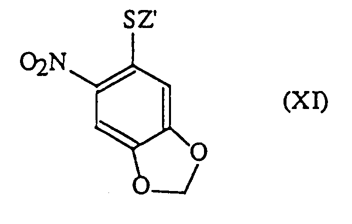 worin Z' einen C₅₋₁₈-Alkylrest, einen Aralkylrest, worin der Alkylrest ein C₂₋₆-Rest ist, einen C₁₋₆-Monohydroxyalkyl oder C₂₋₆-Polyhydroxyalkyl- oder einen Aminoalkylrest der Formel darstellt: 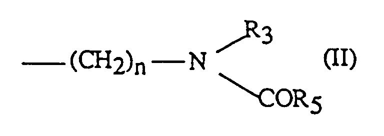 worin gilt:
n ist eine ganze Zahl von 1 bis 6;
R₃ stellt ein Wasserstoffatom oder einen C₁₋₄-Alkylrest, C₁₋₄-Hydroxyalkyl- oder C₂₋₆-Acylrest dar;
R₅ stellt ein Wasserstoffatom oder einen C₁₋₃-Alkylrest dar; insbesondere zur Herstellung der schwefelhaltigen m-Aminophenole der Formel (I) gemäß Anspruch 1.

8. Neue Verbindungen der Formel: 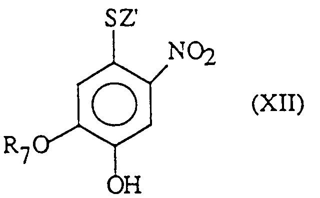 worin gilt:
Z' stellt einen C₁₋₁₈-Alkylrest, einen Aralkylrest, worin der Alkylrest ein C₁₋₆-Rest ist, einen C₁₋₆-Monohydroxyalkyl-, oder C₂₋₆-Polyhydroxyalkylrest, einen Aryl- oder Aminoalkylrest der Formel dar: 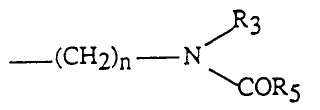 worin gilt:
n ist eine ganze Zahl von 1 bis 6;
R₃ stellt ein Wasserstoffatom oder einen C₁₋₄-Alkyl-, C₁₋₄-Hydroxyalkyl- oder einen C₁₋₆-Acylrest dar;
R₅ stellt ein Wasserstoffatom oder einen C₁₋₃-Alkylrest dar, und worin R₇ einen linearen oder verzweigten C₁₋₄-Alkylrest darstellt,
insbesondere zur Herstellung der schwefelhaltigen m-Aminophenole der Formel (I) gemäß Anspruch 1.

9. Verbindung gemäß Anspruch 8,
dadurch **gekennzeichnet**, daß
es sich um 2-Methoxy-4-methylthio-5-nitrophenol handelt.

10. Zur Färbung keratinischer Fasern und insbesondere menschlicher Haare vorgesehene Verwendung von schwefelhaltigen m-Aminophenolen der allgemeinen Formel: 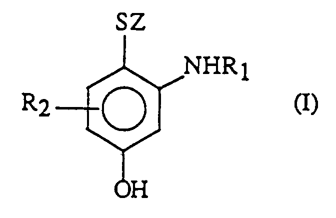 worin Z einen C₁₋₁₈-Alkylrest, einen Aralkylrest, worin der Alkylrest ein C₁₋₆-Rest ist, einen C₁₋₆-Monohydroxyalkyl- oder C₂₋₆-Polyhydroxyalkylrest, einen Arylrest und einen Aminoalkylrest der Formel darstellt: 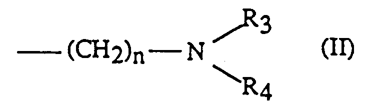 worin gilt:
n ist eine ganze Zahl von 1 bis 6;
R₃ und R₄ stellen, gleich oder verschieden, ein Wasserstoffatom oder einen C₁₋₄-Alkyl-, C₁₋₄-Hydroxyalkyl- oder C₁₋₆-Acylrest dar;
und worin R₁ ein Wasserstoffatom, einen C₁₋₆-Alkyl-, C₁₋₆-Monohydroxyalkyl-, C₂₋₆-Polyhydroxyalkyl-, C₁₋₆-Monocarbamylalkyl-, C₁₋₆-Dicarbamylalkyl-, C₁₋₆-Aminoalkyl-, C₁₋₆-Acylamino-C₁₋₄-alkyl-, C₂₋₆-Carbalkoxy-C₁₋₄-alkyl-, Carbamyl- oder einen C₁₋₆-Monoalkylcarbamylrest und
R₂ ein Wasserstoffatom, einen C₁₋₄-Alkyl-, C₁₋₄-Monohydroxyalkyl- oder einen C₁₋₄-Alkoxyrest darstellen.

11. Färbezusammensetzung für keratinische Fasern und insbesondere die menschlichen Haare,
dadurch **gekennzeichnet**, daß
sie in einem zur Färbung geeigneten Milieu mindestens eine Oxidationsfarbstoff-Vorstufenverbindung vom ortho- und/oder para-Typ und mindestens, als Kuppler, ein schwefelhaltiges m-Aminophenol der Formel : 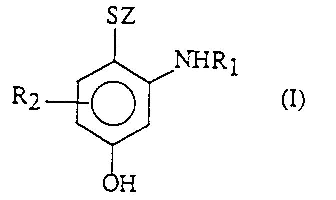 worin Z einen C₁₋₁₈-Alkylrest, einen Aralkylrest, worin der Alkylrest ein C₁₋₆-Rest ist, einen C₁₋₆-Monohydroxyalkyl- oder C₂₋₆-Polyhydroxyalkylrest, einen Arylrest und einen Aminoalkylrest der Formel darstellt: 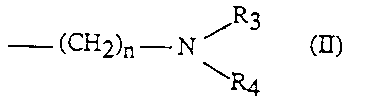 worin gilt:
n ist eine ganze Zahl von 1 bis 6;
R₃ und R₄ stellen, gleich oder verschieden, ein Wasserstoffatom oder einen C₁₋₄-Alkyl-, C₁₋₄-Hydroxyalkyl- oder C₁₋₆-Acylrest dar;
und worin R₁ ein Wasserstoffatom, einen C₁₋₆-Alkyl-, C₁₋₆-Monohydroxyalkyl-, C₂₋₆-Polyhydroxyalkyl-, C₁₋₆-Monocarbamylalkyl-, C₁₋₆-Dicarbamylalkyl-, C₁₋₆-Aminoalkyl-, C₁₋₆-Acylamino-C₁₋₄-alkyl-, C₂₋₆-Carbalkoxy-C₁₋₄-alkyl-, Carbamyl- oder einen C₁₋₆-Monoalkylcarbamylrest und
R₂ ein Wasserstoffatom, einen C₁₋₄-Alkyl-, C₁₋₄-Monohydroxyalkyl- oder einen C₁₋₄-Alkoxyrest darstellen, sowie deren entsprechende Salze enthält.

12. Färbezusammensetzung gemäß Anspruch 11,
dadurch **gekennzeichnet**, daß
die Oxidationsfarbstoff-Vorstufenverbindungen vom ortho- oder para-Typ aus p-Phenylendiaminen, p-Aminophenolen, heterozyklischen Vorstufenverbindungen von para-Derivaten des Pyridins, Pyrimidins oder Pyrazols, aus o-Aminophenolen und Bisphenylalkylendiaminen ausgewählt sind.

13. Färbezusammensetzung gemäß einem der Ansprüche 11 oder 12,
dadurch **gekennzeichnet**, daß
die Verbindungen der Formel (I) aus den folgenden Verbindungen:
dem 2-Methoxy-4-methylthio-5-aminophenol,
dem 4-Methylthio-3-aminophenol
oder deren Additionssalzen mit einer Säure
ausgewählt sind.

14. Färbezusammensetzung gemäß einem der Ansprüche 11 bis 13,
dadurch **gekennzeichnet**, daß
sie ausser den Kupplern der Formel (I) weitere Kuppler wie m-Diphenole, m-Phenylendiamine, m-Aminophenole, die sich von denjenigen der Formel (I) unterscheiden, m-Acylaminophenole, m-Ureidophenole, m-Carbalkoxyaminophenole, α-Naphthol, Indolderivate oder Kuppler mit einer aktiven Methylengruppe enthält.

15. Färbezusammensetzung gemäß einem der Ansprüche 11 bis 14,
dadurch **gekennzeichnet**, daß
sie 0,05 bis 3,5 Gew.% des Gesamtgewichts der Zusammensetzung mindestens einer Verbindung der Formel (I) enthält.

16. Färbezusammensetzung gemäß einem der Ansprüche 11 bis 15,
dadurch **gekennzeichnet**, daß
sie 0,3 bis 7 Gew.%, bezogen auf das Gesamtgewicht der Zusammensetzung, Oxidationsfarbstoff-Vorstufenverbindungen vom ortho- und/oder para-Typ und Kuppler enthält.

17. Färbezusammensetzung gemäß einem der Ansprüche 11 bis 16,
dadurch **gekennzeichnet**, daß
sie ausserdem eine Verbindung, ausgewählt aus kationischen, anionischen, nicht-ionischen und amphoteren oberflächenaktiven Mitteln oder deren Mischungen, in Konzentrationen von 0,5 bis 55 Gew.%, bezogen auf das Gesamtgewicht der Zusammensetzung, organische Lösungsmittel in Konzentrationen von 1 bis 40 Gew.%, bezogen auf das Gesamtgewicht der Zusammensetzung, Verdickungsmittel in Konzentrationen von 0,1 bis 5 Gew.%, bezogen auf das Gesamtgewicht der Zusammensetzung, Antioxidantien in Mengenanteilen von 0,05 bis 1,5 Gew.%, bezogen auf das Gesamtgewicht der Zusammensetzung, und Direktfarbstoffe enthält.

18. Färbezusammensetzung gemäß einem der Ansprüche 11 bis 17,
dadurch **gekennzeichnet**, daß
sie einen pH-Wert von 3 bis 10,5 aufweist.

19. Verfahren zur Oxidationsfärbung keratinischer Fasern und insbesondere menschlicher Haare,
dadurch **gekennzeichnet**, daß
man auf die genannten Fasern eine oder mehrere Oxidationsfarbstoff-Vorstufenverbindungen vom ortho- und/oder para-Typ und ein schwefelhaltiges m-Aminophenol der allgemeinen Formel: 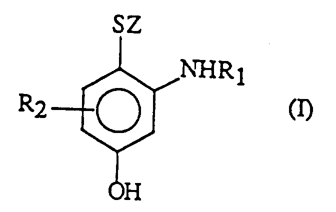 worin Z einen C₁₋₁₈-Alkylrest, einen Aralkylrest, worin der Alkylrest ein C₁₋₆-Rest ist, einen C₁₋₆-Monohydroxyalkyl- oder C₂₋₆-Polyhydroxyalkylrest, einen Arylrest und einen Aminoalkylrest der Formel darstellt: 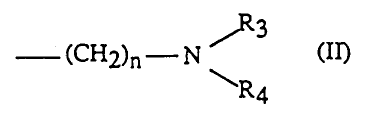 worin gilt:
n ist eine ganze Zahl von 1 bis 6;
R₃ und R₄ stellen, gleich oder verschieden, ein Wasserstoffatom oder einen C₁₋₄-Alkyl-, C₁₋₄-Hydroxyalkyl- oder C₁₋₆-Acylrest dar;
und worin R₁ ein Wasserstoffatom, einen C₁₋₆-Alkyl-, C₁₋₆-Monohydroxyalkyl-, C₂₋₆-Polyhydroxyalkyl-, C₁₋₆-Monocarbamylalkyl-, C₁₋₆-Dicarbamylalkyl-, C₁₋₆-Aminoalkyl-, C₁₋₆-Acylamino-C₁₋₄-alkyl-, C₂₋₆-Carbalkoxy-C₁₋₄-alkyl-, Carbamyl- oder einen C₁₋₆-Monoalkylcarbamylrest und
R₂ ein Wasserstoffatom, einen C₁₋₄-Alkyl-, C₁₋₄-Monohydroxyalkyl- oder einen C₁₋₄-Alkoxyrest darstellen,
und deren Säuresalze, in Gegenwart eines oxidierenden Mittels, aufbringt.

20. Färbeverfahren gemäß Anspruch 19,
dadurch **gekennzeichnet**, daß
man, zum Zeitpunkt der Anwendung, eine Färbezusammensetzung für keratinische Fasern und insbesondere die menschlichen Haare, die, in einem zur Färbung geeigneten Milieu, mindestens eine Oxidationsfarbstoff-Vorstufenverbindung vom ortho- und/oder para-Typ und mindestens, als Kuppler, ein schwefelhaltiges m-Aminophenol der Formel (I) enthält, mit einer oxidierenden Lösung in einer zur Entwicklung der Färbung ausreichenden Menge vermischt, dann die erhaltene Mischung auf die keratinischen Fasern und insbesondere die menschlichen Haare aufbringt, wobei der pH-Wert der Mischung 2 bis 13 beträgt, daß man die Mischung 10 bis 40 und vorzugsweise 15 bis 30 Minuten lang verweilen läßt, die Haare spült, sie unter Schamponieren wäscht, erneut spült und dann trocknet.
